# EUROPEAN PATENT APPLICATION

(11) **EP 4 813 413 A1**
(43) Date of publication of application: **30.09.2026**
(21) Application number: 25166493.4
(22) Date of filing: 26.03.2025
(51) Int. Cl.: A61M 5/14

(54) **HOLDER FOR SYRINGE-BASED ENTERAL FEEDING ASSEMBLIES**

(71) Applicant: Dignified Solutions, 9000 Aalborg (DK)
(72) Inventor: Rønnow, Stephan, 9000 Aalborg (DK)
(74) Representative: Larsen & Birkeholm A/S

(57) **Abstract**

The present invention relates to a holder specifically designed for syringe-based enteral feeding assemblies.

## Description

### Technical field of the invention

The present invention relates to the field of enteral feeding assemblies.

### Background of the invention

Enteral feeding assemblies that employ syringes for holding and administering food are critical in medical settings, particularly for delivering nutrition directly to a patient's stomach or intestine. This method is indispensable for patients who are unable to consume food orally yet possess a functioning digestive system. In setups where gravity assists in feeding without the use of a traditional feeding bag, large-volume syringes, typically ranging from 10 ml to 150 ml (with capacities between 10 ml and 20 ml specifically used for neonatal care) are utilized. These syringes are mounted on a stand that positions them above the patient to leverage gravity, facilitating the flow of nutrition.

The syringes are filled with a prescribed liquid nutrition and strategically placed in an elevated position to utilize gravitational forces. The administration of the formula can be regulated either through a plunger equipped with a slow-release mechanism or via manual adjustment, ensuring a controlled and steady delivery of the nutritional formula into the feeding tube.

The feeding tube, a critical component of the assembly, is connected to the syringe via a secure interface that prevents leaks and contamination. This tubing is typically made from medical-grade materials that are resistant to degradation by the nutritional formula and body fluids. It is designed to be flexible yet sturdy enough to ensure an unobstructed flow and minimize the risk of kinks that could impede the delivery of nutrition. The tubing can extend several feet, depending on the setup, allowing for patient mobility and comfort. The distal end of the tubing is carefully inserted into the patient's stomach or intestine, and its placement may be confirmed by medical imaging techniques to ensure correct positioning.

To maintain the integrity and safety of the feeding system, regular cleaning and sterilization of the syringe and tubing are imperative. This is crucial not only to prevent infection but also to ensure the system remains free from any residues that could clog the tubing. In some cases, particularly in high-risk environments or when quick turnaround is needed, syringes may be designed for single use, thus eliminating the need for cleaning and reducing the risk of cross-contamination.

An innovative solution that enhances the usability and effectiveness of this feeding system is the FreeArm Tube Feeding Assistant by FreeArm Care. This device securely holds the syringe, thereby freeing up the hands of caregivers. This support allows caregivers to engage in other tasks or to interact more intimately with the patient, thus improving the quality of care. The FreeArm system exemplifies how auxiliary devices can integrate into enteral feeding setups to improve operational efficiency and enhance patient care.

### Object of the Invention

The objective of the present invention is to provide a holder that simplifies the setup and operation of syringe-based enteral feeding systems, making them easy to use with a single hand.

### Summary of the Invention

The present invention relates to a holder specifically designed for syringe-based enteral feeding assemblies. This invention addresses the need for a simplified and efficient means to secure a syringe during enteral feeding procedures, facilitating use with a single hand. A primary objective of this invention is to provide a holder that enhances usability and stability for caregivers during feeding sessions.

The invention comprises a holder equipped with a specially designed base. The base features an upper face, a lower face, and one or more side faces. A key feature of this holder is the incorporation of a recess in one or more of these faces, meticulously shaped to receive and secure a syringe's finger flange. This recess is specifically adapted to hold the finger flange firmly in place, thereby ensuring the syringe remains stable and upright when positioned within the holder. This design facilitates ease of use and increases safety by minimizing the risk of syringe displacement during operation.

A first aspect relates to a holder for a syringe-based enteral feeding assembly, the holder comprising:
- a base with an upper face, a lower face, and one or more side faces; and
- a suspension element connected to the base;
wherein the base comprises a recess formed in one or more of its faces, said recess adapted for receiving a syringe's finger flange, and wherein the recess is adapted to secure the finger flange in place, thereby stabilizing the syringe when positioned in the recess.

As used in the specification and the appended claims, the singular forms "a", "an", and "the" include plural referents unless the context clearly dictates otherwise. Ranges may be expressed herein as from "about" or "approximately" one particular value and/or to "about" or "approximately" another particular value. When such a range is expressed, another embodiment includes from the one particular value and/or to the other particular value. Similarly, when values are expressed as approximations, by use of the antecedent "about", it will be understood that the particular value forms another embodiment.

It should be noted that embodiments and features described in the context of one of the aspects of the present invention also apply to the other aspects of the invention.

### Brief description of the figures

Figure 1 is a schematic side view of a holder in accordance with various embodiments of the invention.
Figure 2 is a schematic bottom perspective view of a holder in accordance with various embodiments of the invention.
Figures 3-7 are schematic top perspective views of different holders in accordance with various embodiments of the invention.

### References

- 100: Holder
- 110: Base
- 111: Upper face
- 112: Lower face
- 113: Side face
- 114: Recess
- 115: Seat
- 116: Inlet
- 117: Lid
- 118: Protrusion
- 120: Suspension element

### Detailed Description of the Invention

In the present context, the term "in general" when used when mentioning a feature relating to the present invention, it must be understood that the feature may be used with all embodiments of the invention, even if the mentioning is made in the detailed part of the document.

A first aspect relates to a holder for a syringe-based enteral feeding assembly, the holder comprising:
- a base with an upper face, a lower face, and one or more side faces;
wherein the base comprises a recess formed in one or more of its faces, said recess adapted for receiving a syringe's finger flange, and wherein the recess is adapted to secure the finger flange in place, thereby stabilizing the syringe when positioned in the recess.

A second aspect relates to a holder for a syringe-based enteral feeding assembly, the holder comprising:
- a base with an upper face, a lower face, and one or more side faces; and
- a suspension element connected to the base;
wherein the base comprises a recess formed in one or more of its faces, said recess adapted for receiving a syringe's finger flange, and wherein the recess is adapted to secure the finger flange in place, thereby stabilizing the syringe when positioned in the recess.

In a syringe-based enteral feeding assembly, the syringe is a key component designed to deliver nutrition directly to a patient's gastrointestinal tract, typically when they are unable to eat by mouth but have a functioning digestive system.

The syringe typically consists of a cylindrical barrel that holds the nutritional formula. This barrel is usually made from a transparent or translucent material, allowing caregivers to easily monitor the contents and volume. Measurements are marked along the side of the barrel to facilitate precise dosage control.

At one end of the barrel, there is a nozzle or outlet. This end can be fitted with various types of connectors or adapters that attach to a feeding tube, leading directly to the patient's stomach or intestine. The nozzle is designed to ensure a secure and leak-proof connection to the feeding tube.

The opposite end of the barrel is open and may or may not include a plunger. In traditional syringe designs, a plunger is used to manually push the nutritional formula through the barrel and into the feeding tube. The plunger typically has a rubber or silicone gasket that forms a tight seal against the inner walls of the barrel to prevent backflow and ensure smooth operation.

However, in some designs of syringe-based enteral feeding assemblies, the use of a plunger is optional. These syringes are designed for passive feeding setups where gravity or a specialized feeding pump can be used to administer the formula. In such cases, the syringe acts more like a reservoir that is mounted in an elevated position or connected to a feeding pump. The gravitational force or pump pressure moves the formula through the feeding tube without the need for manual plunger intervention.

Finger flanges are critical ergonomic features located at the back end of a syringe's barrel, where the plunger is inserted. These flanges are designed to facilitate the manual operation of the syringe, providing a place for the user's fingers to rest and apply force when administering the contents of the syringe.

Typically, finger flanges are extended wings or tabs that protrude laterally from the syringe barrel. They are strategically positioned to offer maximum leverage and comfort during the injection or feeding process. This design allows for a secure grip, which is essential for smooth and controlled plunger movement, especially during precise dosing or slow administration of fluids.

Finger flanges are usually made from the same material as the syringe barrel, i.e., commonly a type of durable, medical-grade plastic. This ensures that the flanges are sturdy yet lightweight, adding minimal bulk to the syringe while providing sufficient strength for repeated use. The integration of the flanges with the barrel is seamless, often formed as a single piece during the manufacturing process, which enhances the overall integrity and hygiene of the syringe.

In the context of syringe-based enteral feeding assemblies, finger flanges play an important role. They allow caregivers to comfortably and securely hold the syringe during feeding, facilitating the manual delivery of nutritional formulas into the feeding tube. This is particularly important when precision and control over the feeding rate are necessary to accommodate the patient's digestive tolerance.

The finger flanges are in the present invention utilized in a new manner. Here, the flanges serve as stabilization points when mounting the syringe in the base 110 of the holder 100.

The base 110 of the holder 100 for syringe-based enteral feeding assemblies is meticulously designed to optimize both functionality and user experience, typically constructed from a polymeric material suitable for medical applications. Such materials are well-known within the art and may comprise polypropylene (PP), polycarbonate (PC), polyethylene (PE), polyetheretherketone (PEEK), polytetrafluoroethylene (PTFE), polyurethanes (PU) (such as thermoplastic polyurethane (TPU)), silicone, acrylonitrile, and butadiene styrene (ABS). This material choice ensures durability and chemical resistance, facilitating the use of advanced manufacturing techniques.

Through injection moulding, molten polymer is precisely injected into a mould to form the base and its integral features, including the specialized recesses. Alternatively, CNC machining offers the capability to carve out complex geometries tailored to precise specifications, particularly useful for creating custom features on the base.

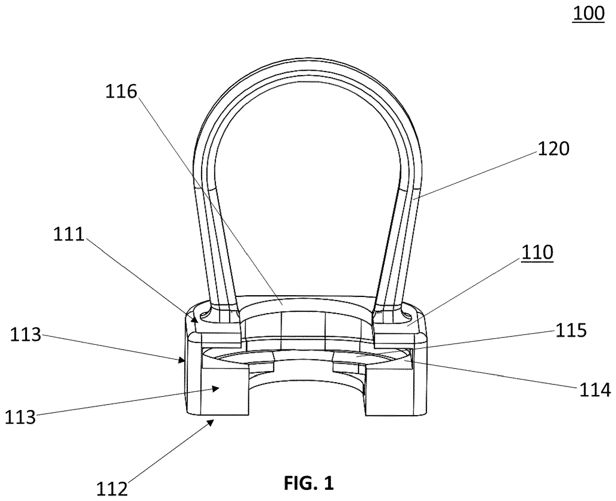

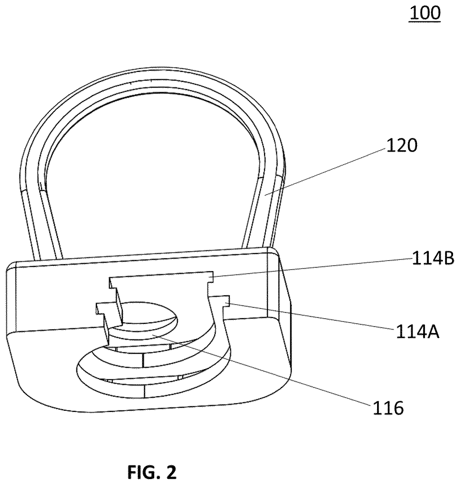

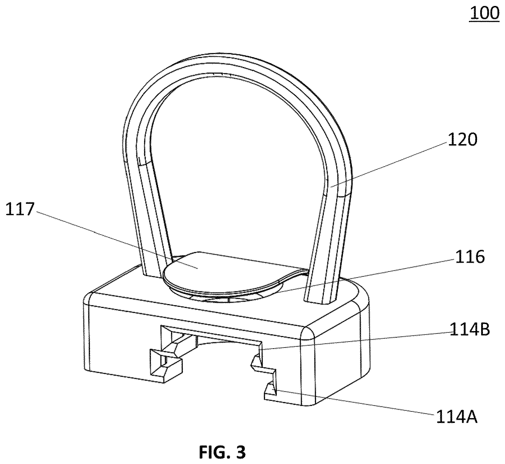

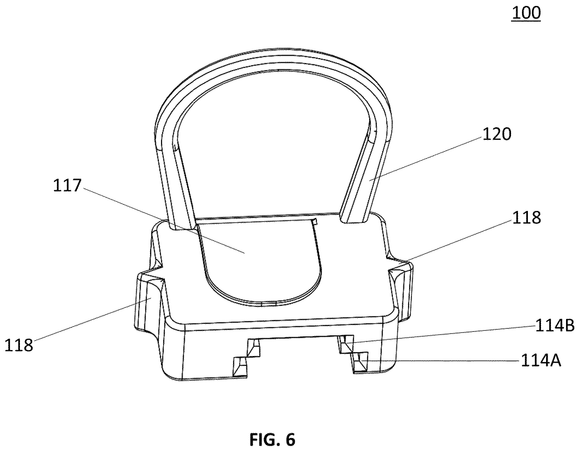

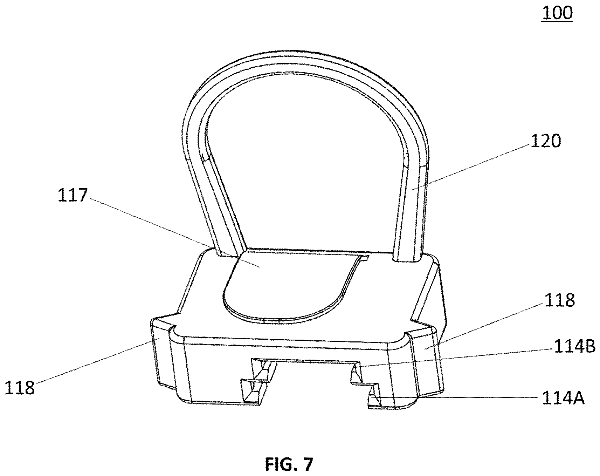

Central to the holder's functionality is the recess 114, designed specifically to securely accommodate the syringe's finger flange. The design of the base 110 can vary, featuring either a single recess 114 (see Figure 1) or multiple recesses 114A, 114B (see Figures 2-3, and 6-7) to support different syringe sizes.

In configurations with a single recess 114, an integrally formed seat 115 (see Figure 1) may engage with the syringe's finger flanges, ensuring that the syringe is retained securely and stably when introduced.

For broader compatibility, the base 110 may also include multiple recesses 114A, 114B of varying sizes, arranged to accommodate syringes with different finger flanges. Some designs optimize space by arranging these recesses 114A, 114B vertically (see Figures 2-3, and 6-7), enhancing both accessibility and efficiency.

The recess(es) 114 may feature a curved configuration (see Figure 2) to enhance retention, allowing the syringe's finger flanges to be turned within the recess 114. This design prevents the syringe from falling out, thereby adding an extra layer of security during use.

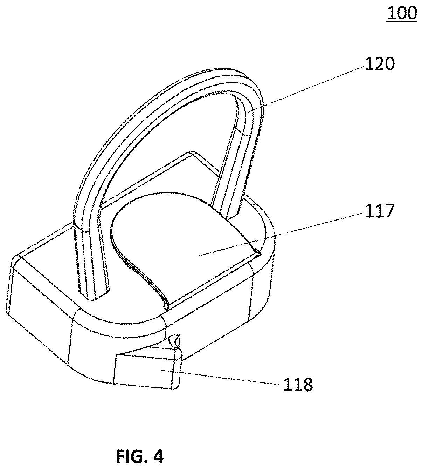

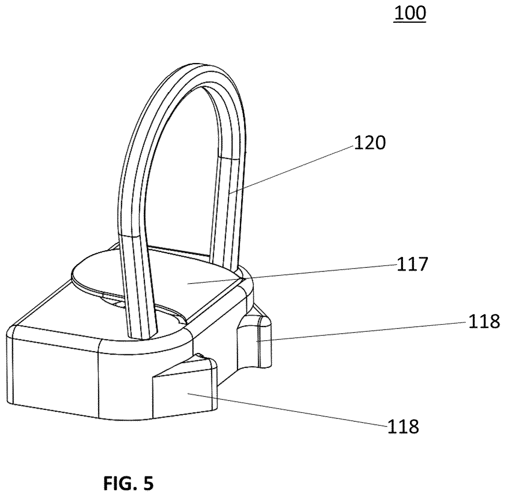

In general, the side faces 113 of the base 110 may be equipped with grip enhancements, such as textured grips, ridges, grooves, soft-touch materials, or one or more (e.g., two) protrusions 118 (see Figures 4-7), e.g., configured as finger counter holds, which all provide the user with a better grip. These ergonomic features are preferred for ensuring the holder 110 can be operated comfortably and securely, even with one hand.

The holder is preferably designed with an inlet 116 (see Figures 1-3) directly connected to the recess 114, enabling the back-end refilling of the syringe. This feature allows for the easy replenishment of medications or nutritional formulas without needing to remove the syringe, thereby enhancing operational efficiency. A lid 117 (see Figures 4-7) may also be integrated to engage with this inlet 116, sealing the back end of the syringe's barrel to maintain sterility and prevent contamination when not in use or during refilling.

The integration of these features into the base of the holder not only maximizes its practical utility but also enhances its usability, making it exceptionally user-friendly in diverse medical environments. This design significantly improves the efficiency and safety of syringe-based enteral feeding, catering to the essential needs of caregivers.

In general, the holder 100 may also comprise a suspension element 120, here embodied as a loop. In general, the term "suspension element" is here to be understood as a component or structure of the holder designed and utilized to support, attach, or hang the base from another object, such as a hospital bed, an infusion bag hanger, or similar support structures, or a necklace (a caregiver may e.g., hold the child that is fed, while the holder hangs in the caregiver's necklace). This element facilitates vertical suspension and stability when in use and includes, but is not limited to, features such as loops, rings, hooks, or clasps. These features enable the object to interact with various types of supporting structures commonly found in medical environments, ensuring the object remains securely suspended and readily accessible.

In one or more embodiments, the base includes a single recess formed in one of its faces; wherein a seat is integrally formed within the base and is in direct connection with the recess; wherein the seat is specifically configured to receive and engage with the finger flanges of the syringe; and wherein when the syringe is introduced into the recess, the seat is configured such that the syringe's finger flanges will drop into and be retained by the seat, thereby securing the syringe in a stable orientation.

In one or more embodiments, the base comprises multiple recesses formed in one or more of its faces, each recess adapted for receiving a syringe's finger flange, wherein each recess is of a different size to accommodate syringes with finger flanges of varying dimensions.

In one or more embodiments, there are two recesses arranged one above the other on one or more of the faces of the base.

In one or more embodiments, the recess(es) are adapted for receiving the syringe's finger flange as well as the end part of the syringe's barrel on which the finger flanges are formed.

In one or more embodiments, the recess(es) are of a curved configuration configured to allow the finger flanges of a syringe to be turned within the recess, thereby preventing the syringe from falling out of the recess when in place.

In one or more embodiments, the base further comprises an inlet being in direct connection, such as in fluid communication, with the recess(es), thereby allowing a mounted syringe to be filled via its back end and through said inlet.

In one or more embodiments, the base further comprises a lid configured to engage with the inlet to form a seal, thereby closing the back end of the syringe's barrel.

In one or more embodiments, the suspension element is configured as a loop, ring, hook, or clasp.

In one or more embodiments, one or more of the base's side faces are equipped with grip enhancements, such as textured grips, ridges, grooves, soft-touch materials, or one or more protrusions.

## Claims

1. A holder (100) for a syringe-based enteral feeding assembly, the holder comprising:
- a base (110) with an upper face (111), a lower face (112), and one or more side faces (113);
wherein the base (110) comprises a recess (114) formed in one or more of its faces (111, 112, 113), said recess (114) adapted for receiving a syringe's finger flange, and wherein the recess (114) is adapted to secure the finger flange in place, thereby stabilizing the syringe when positioned in the recess (114).

2. The holder (100) according to claim 1, wherein the base (110) includes a single recess (114) formed in one of its faces (111, 112, 113); wherein a seat (115) is integrally formed within the base (110) and is in direct connection with the recess (114); wherein the seat (115) is specifically configured to receive and engage with the finger flanges of the syringe; and wherein when the syringe is introduced into the recess (114), the seat (115) is configured such that the syringe's finger flanges will drop into and be retained by the seat (115), thereby securing the syringe in a stable orientation.

3. The holder (100) according to claim 1, wherein the base (110) comprises multiple recesses (114A, 114B) formed in one or more of its faces (111, 112, 113), each recess (114A, 114B) adapted for receiving a syringe's finger flange, wherein each recess (114A, 114B) is of a different size to accommodate syringes with finger flanges of varying dimensions.

4. The holder (100) according to claim 3, wherein there are two recesses (114A, 114B) arranged one above the other on one or more of the faces (111, 112, 113) of the base (110).

5. The holder (100) according to any one of the claims 1-4, wherein the recess(es) (114) are adapted for receiving the syringe's finger flange as well as the end part of the syringe's barrel on which the finger flanges are formed.

6. The holder (100) according to any one of the claims 1-5, wherein the recess(es) (114) are of a curved configuration configured to allow the finger flanges of a syringe to be turned within the recess (114), thereby preventing the syringe from falling out of the recess (114) when in place.

7. The holder (100) according to any one of the claims 1-6, wherein the base (110) further comprises an inlet (116) being in direct connection, such as in fluid communication, with the recess(es) (114), thereby allowing a mounted syringe to be filled via its back end and through said inlet (116).

8. The holder (100) according to claim 7, wherein the base (110) further comprises a lid (117) configured to engage with the inlet (116) to form a seal, thereby closing the back end of the syringe's barrel.

9. The holder (100) according to any one of the claims 1-8, further comprising a suspension element (120) connected to the base (110).

10. The holder (100) according to any one of the claims 1-9, wherein one or more of the base's side faces (113) are equipped with grip enhancements, such as textured grips, ridges, grooves, soft-touch materials, or one or more protrusions (118).
